# EUROPEAN PATENT APPLICATION

(11) **EP 4 696 215 A1**
(43) Date of publication of application: **18.02.2026**
(21) Application number: 24806251.5
(22) Date of filing: 15.04.2024
(51) Int. Cl.: A61B 1/313

(54) **LAPAROSCOPE DRIVE SYSTEM, LAPAROSCOPE INSTALLATION DETECTION METHOD, AND SURGICAL ROBOT**

(30) Priority: 15.05.2023 CN 202310543051
(71) Applicant: Ronovo (Shanghai) Medical Science and Technology Ltd., Shanghai 201102 (CN)
(72) Inventor: SU, Zhiqiang, Shanghai 201102 (CN)
(74) Representative: Cabinet Beaumont
(86) International application number: PCT/CN2024/087765
(87) International publication number: WO 2024/234894

(57) **Abstract**

Provided are a laparoscope drive system, a laparoscope mounting detection method, and a surgical robot, which belong to the technical field of medical devices. The laparoscope drive system includes a base plate, a drive unit, a sterile barrier, a trigger assembly, and a state detector. The sterile barrier is configured for mounting a laparoscope; the drive unit drives the laparoscope to rotate via the sterile barrier; the trigger assembly is disposed in the sterile barrier and selectively abuts against the laparoscope, and the trigger assembly has a first state and a second state; and the state detector is configured to detect state information of the trigger assembly to determine a mounted state of the laparoscope.

## Description

This application claims priority to Chinese Patent Application No. 202310543051.8 filed with the China National Intellectual Property Administration (CNIPA) on May 15, 2023, the disclosure of which is incorporated herein by reference in its entirety.

### TECHNICAL FIELD

The present application relates to the technical field of medical devices, for example, a laparoscope drive system, a laparoscope mounting detection method, and a surgical robot.

### BACKGROUND

Due to the applications of surgical robots in the medical field, many problems that are difficult to solve through manual operations have been solved. For example, a laparoscope on a surgical robot can extend into a patient's body to provide a surgeon with a visual field, making it convenient for the surgeon to perform the corresponding operations.

In the related art, the laparoscope on the surgical robot is disposed at an end of a robotic arm and connected to a drive system in the robotic arm so that the laparoscope is powered by the drive system to perform the corresponding actions, such as rotation or translation, to smoothly provide a field for the surgeon. Since the mounting of the laparoscope requires a sterile environment, a sterile barrier is provided between the laparoscope and the drive system. During mounting, the laparoscope is required to extend into the sterile barrier and be fixed by an internal snap-fit structure. To ensure that the laparoscope is mounted stably, a detector, such as a microswitch, is generally added to the original structure.

However, when the microswitch is used, if the laparoscope is in direct contact with the microswitch, not only is the service life of the microswitch affected due to the movement and rotation of the laparoscope, but also a mounting space of the laparoscope is occupied, affecting an operating route of the laparoscope. If a dedicated mechanical structure is added to trigger the microswitch, although an occupied axial space can be reduced, the overall conversion efficiency of a rotation structure decreases and the friction increases, easily causing the stuttering of the laparoscope during insertion and removal and reducing the overall usability and operating experience of the surgical robot.

### SUMMARY

The present application provides a laparoscope drive system, a laparoscope mounting detection method, and a surgical robot, solving the problems of poor overall usability and operating experience of a surgical robot since the detection of a mounting situation of a laparoscope with a detector such as a microswitch during mounting of the laparoscope affects the normal use of the laparoscope in the related art.

In a first aspect, the present application provides a laparoscope drive system. The laparoscope drive system includes a base plate; a drive unit disposed on the base plate and configured to drive a laparoscope to rotate via a sterile barrier; a trigger assembly disposed in the sterile barrier and selectively abutting against the laparoscope, where the trigger assembly has a first state indicating that the laparoscope is mounted in place and a second state indicating that the laparoscope is not mounted in place; and a state detector disposed in the base plate and configured to detect state information of the trigger assembly to determine a mounted state of the laparoscope.

In a second aspect, the present application provides a laparoscope mounting detection method. The laparoscope mounting detection method includes: controlling a state detector to start to acquire state information of a trigger assembly, in response to the state information satisfying a preset condition, determining that the trigger assembly is in a first state when a laparoscope is mounted in place, and in response to the state information not satisfying the preset condition, determining that the trigger assembly is in a second state when the laparoscope is not mounted in place.

In a third aspect, the present application provides a surgical robot. The surgical robot includes a robotic arm, a laparoscope, and the laparoscope drive system according to the first aspect. The laparoscope drive system is disposed on the robotic arm and connected to the laparoscope.

### BRIEF DESCRIPTION OF DRAWINGS

FIG. 1 is an exploded view of a laparoscope drive system according to some embodiments of the present application.
FIG. 2 is an exploded view of a sterile barrier and a trigger assembly of a laparoscope drive system according to some embodiments of the present application.
FIG. 3 is a sectional view of a laparoscope drive system without a laparoscope mounted in a sterile barrier according to some embodiments of the present application.
FIG. 4 is a sectional view of a laparoscope drive system with a laparoscope mounted in a sterile barrier according to some embodiments of the present application.
FIG. 5 is a structural view of a base plate of a laparoscope drive system according to some embodiments of the present application.
FIG. 6 is a sectional view of a laparoscope drive system with a laparoscope mounted in a trigger sleeve according to some embodiments of the present application.
FIG. 7 is a sectional view of a laparoscope drive system without a laparoscope mounted in a trigger sleeve according to some embodiments of the present application.
FIG. 8 is a schematic view of operation of a surgical robot according to some embodiments of the present application.

### Reference list

- 1: base plate
- 10: arc-shaped mounting strip
- 2: drive unit
- 20: outer housing
- 21: inner housing
- 3: sterile barrier
- 30: first sterile shell
- 31: second sterile shell
- 310: sterile plate
- 311: sterile sleeve
- 312: top ring
- 313: detection gap
- 4: state detector
- 40: ray sensor
- 41: detection circuit board
- 5: trigger assembly
- 50: trigger sleeve
- 51: reset member
- 501: first collar
- 502: second collar
- 6: laparoscopy
- 7: robotic arm

### DETAILED DESCRIPTION

In the description of the present application, terms "joined", "connected", and "fixed" are to be understood in a broad sense unless otherwise expressly specified and limited. For example, the term "connected" may refer to "fixedly connected", "detachably connected", or "integrated", may refer to "mechanically connected" or "electrically connected", may refer to "connected directly" or "connected indirectly through an intermediary", or may refer to "connected inside two components" or "an interaction relation between two components". For those of ordinary skill in the art, meanings of the preceding terms in the present application may be understood according to situations.

In the present application, unless otherwise expressly specified and limited, when a first feature is described as "on" or "below" a second feature, the first feature and the second feature may be in direct contact or be in contact via another feature between the two features instead of being in direct contact. Moreover, when the first feature is described as "on", "above", or "over" the second feature, the first feature is right on, above, or over the second feature, the first feature is obliquely on, above, or over the second feature, or the first feature is simply at a higher level than the second feature. When the first feature is described as "under", "below", or "underneath" the second feature, the first feature is right under, below, or underneath the second feature, the first feature is obliquely under, below, or underneath the second feature, or the first feature is simply at a lower level than the second feature.

In the description of this embodiment, orientations or position relations indicated by terms such as "above", "below", and "right" are based on the drawings. These orientations or position relations are intended only to facilitate description and simplify operations and not to indicate or imply that a device or element referred to must have such particular orientations or must be configured or operated in such particular orientations. Thus, these orientations or position relations are not to be construed as limiting the present application. Additionally, terms "first" and "second" are used only for the distinguishing purpose and have no special meanings.

The present application provides a laparoscope drive system, a laparoscope mounting detection method, and a surgical robot. The surgical robot is connected to a laparoscope via the laparoscope drive system, and the laparoscope mounting detection method adopts a non-contact detection manner to determine whether the laparoscope is mounted in place according to the intensity of the received reflected infrared light so that during detection, a detection element is not required to be in direct contact with the laparoscope and does not occupy a mounting space of the laparoscope. The laparoscope is still driven by a drive element via a sterile barrier, ensuring that the conversion efficiency of a rotation structure is not reduced and avoiding an increase in friction. Thus, the laparoscope is prevented from stuttering during insertion and removal.

Moreover, in the related art, a magnetic sensor or a photoelectric sensor is used as the detection element. During use, the magnetic sensor is prone to a false trigger during mounting of the laparoscope because the whole robotic arm has relatively many structures made of metal materials, and the addition of a dedicated magnet to the laparoscope results in an increase in the production cost. The photoelectric sensor is mainly applied to the detection of static or dynamic parts on planes and has relatively low detection accuracy for the laparoscope and its corresponding mounting structure, both of which are rotary bodies.

In the present application, the intensity of the reflected infrared light is detected so that whether the laparoscope is mounted in place is detected. The intensity of the reflected infrared light may change according to different distances, coatings, or irradiated materials so that during detection, different intensities of infrared light can be directly reflected due to structural characteristics of the sterile barrier. A rotary body can also gain a relatively strong or weak reflective property under the action of a coating or material. Thus, the detection accuracy in this detection process is higher than those of the magnetic sensor and the photoelectric sensor, which is conducive to ensuring the usability and operating experience of the surgical robot.

### Embodiment one

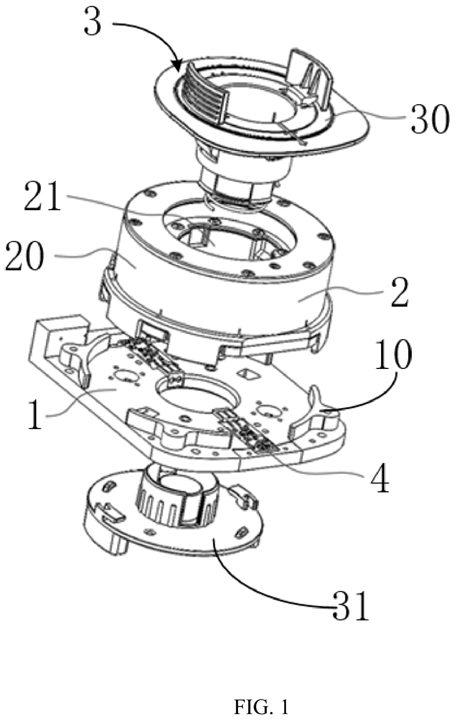

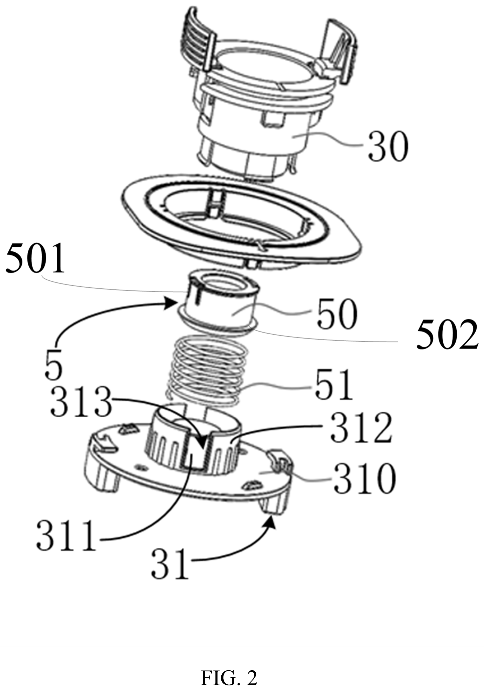

Referring to FIGS. 1 and 2, the present application provides a laparoscope drive system, and the laparoscope drive system includes a base plate 1, a drive unit 2, a state detector 4, and a trigger assembly 5. The drive unit 2 is disposed on the base plate 1 and configured to drive a laparoscope 6 to rotate via a sterile barrier 3. The trigger assembly 5 is disposed in the sterile barrier 3 and selectively abuts against the laparoscope, and the trigger assembly 5 has a first state indicating that the laparoscope is mounted in place and a second state indicating that the laparoscope is not mounted in place. The state detector 4 is disposed in the base plate 1 and configured to detect state information of the trigger assembly to determine a mounted state of the laparoscope.

For example, the base plate 1 is plate-shaped, and multiple arc-shaped mounting strips 10 may be provided on a top surface of the base plate 1 and circumferentially distributed at intervals, where the multiple arc-shaped mounting strips 10 surround a space for mounting the drive unit 2. The whole drive unit 2 is a rotary body, and an outer sidewall of the drive unit 2 is fitted to the arc-shaped mounting strips 10. The drive unit 2 includes an outer housing 20 and an inner housing 21 rotatably connected, via a bearing, inside the outer housing 20, and the sterile barrier 3 is inserted into the inner housing 21. The sterile barrier 3 and the drive unit 2 are two independent devices in a split configuration. When the laparoscope 6 needs to be mounted, the sterile barrier 3 is mounted into the drive unit 2. After the laparoscope 6 is detached, the sterile barrier 3 may also be detached from the drive unit 2 to be stored or replaced separately.

The sterile barrier 3 may be divided into two hollow parts, one part is inserted into the drive unit 2 from the upper side, and the other part is inserted into the drive unit 2 through the base plate 1. The two parts are butted to form a sterile mounting cavity for mounting the laparoscope 6.

The trigger assembly 5 is disposed in the sterile barrier 3 and movably connected to the sterile barrier 3. The trigger assembly 5 cannot be detached from the sterile barrier 3 so that the sterile barrier 3 and the trigger assembly 5 form a complete structure, and after the mounting of the sterile barrier 3 is completed in the drive unit 2, the trigger assembly 5 is mounted in place, effectively reducing the mounting difficulty of the laparoscope drive system. Moreover, the whole sterile barrier 3 including the trigger assembly 5 is a rotary body. After the laparoscope is mounted in place, when the drive unit 2 drives the laparoscope to rotate, the trigger assembly 5 may rotate with the laparoscope or may remain stationary. In either case, the state information detected by the state detector 4 has no difference, effectively ensuring the safety of the laparoscope during use.

The trigger assembly 5 can abut against the laparoscope inserted into the sterile mounting cavity. With the insertion of the laparoscope, the trigger assembly 5 can gradually move downward to change from the second state to the first state. A structure such as a trench is defined on the top surface of the base plate 1, and the state detector 4 is disposed in the trench. Moreover, the state detector 4 can correspond to the sterile barrier 3 in the drive unit 2 so that the state detector 4 can correspond to the trigger assembly 5 in the first state.

The design of the trench can not only provide a mounting space for the state detector 4 but also prevent the state detector 4 from being damaged by the sterile barrier 3 and the drive unit 2 when the sterile barrier 3 and the drive unit 2 are mounted to the base plate 1, thereby protecting the state detector 4. Meanwhile, the state detector 4 is embedded in the trench so that the overall size of the laparoscope drive system does not change due to the state detector 4, and the overall axial dimension of the laparoscope drive system remains within a reasonable range, reducing a possibility that a route of the laparoscope is occupied.

When the laparoscope drive system is used for driving the laparoscope to move, the sterile barrier 3 is mounted into the drive unit 2, and then the laparoscope is inserted into the sterile mounting cavity. As the laparoscope is inserted, the trigger assembly 5 gradually changes from the second state to the first state. When the state detector 4 detects that the trigger assembly 5 is in the first state, it indicates that the laparoscope is mounted in place. After the laparoscope is mounted in place, the drive unit 2 drives the sterile barrier 3 to rotate so that the laparoscope can be driven to rotate synchronously. Thus, the state detector 4 can determine the state information of the trigger assembly 5 by detecting the trigger assembly 5 and can specify whether the laparoscope is mounted in place without being in direct contact with the laparoscope. Moreover, the trigger assembly 5 is disposed in the sterile barrier 3 and does not occupy the mounting space of the laparoscope. Additionally, the laparoscope is still driven via the sterile barrier 3, the conversion efficiency of a rotation structure is not affected by the trigger assembly 5 and the state detector 4, and a possibility of an increase in friction can be effectively reduced, preventing the laparoscope from stuttering during insertion and removal.

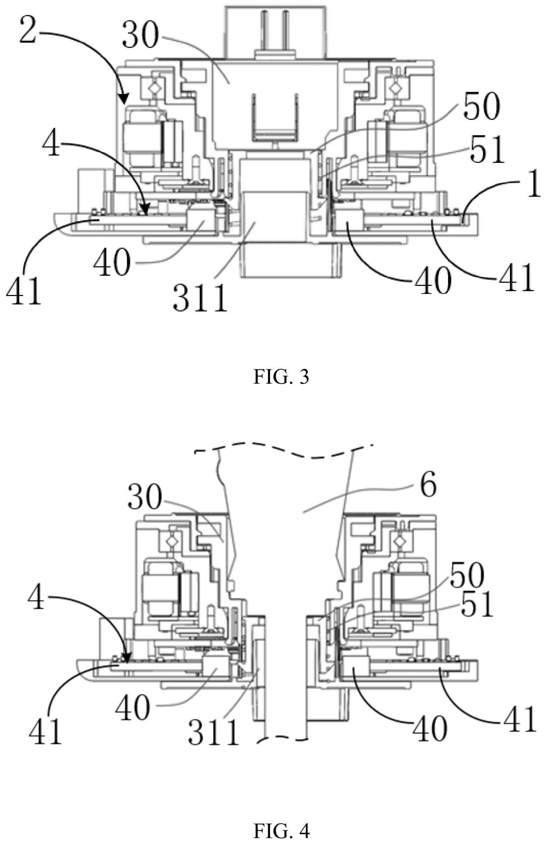

Referring to FIGS. 3 and 4, in some embodiments of the present application, the state detector 4 is capable of generating a ray and receiving a ray reflected by the sterile barrier 3 or the trigger assembly 5, for example, receiving a ray reflected by a trigger sleeve 50, to detect the state information of the trigger assembly 5 according to an intensity of the reflected ray. In this embodiment, the ray generated and received by the state detector 4 is infrared light. In other embodiments, other applicable rays may also be used.

For example, the state detector 4 includes a ray sensor 40, a detection circuit board 41, an amplifier, and a comparator. The ray sensor 40 is configured to emit infrared light and receive reflected infrared light; the detection circuit board 41 is communicatively connected to the ray sensor 40 to convert the reflected infrared light into an analog signal; the amplifier is disposed on the detection circuit board 41 and configured to amplify the analog signal; and the comparator is disposed on the detection circuit board 41 and communicatively connected to the amplifier to perform comparative processing on the amplified analog signal to generate the corresponding state information. For example, for the compositions and control logics of the ray sensor 40, the detection circuit board 41, the amplifier, and the comparator, reference may be made to the related art, and the details are not repeated here. The ray sensor 40 may emit other rays in addition to the infrared light.

In the first state, the infrared light emitted by the ray sensor 40 is directly irradiated on the trigger assembly 5 to form the reflected infrared light, the ray sensor 40 receives the reflected infrared light, the detection circuit board 41 may convert an intensity of the received infrared light into the analog signal, the amplifier amplifies the analog signal and transmits the amplified analog signal to the comparator, and the comparator compares the amplified analog signal with a preset intensity threshold, where a signal exceeding the intensity threshold is set to 1 and corresponds to the first state of the trigger assembly 5.

In the second state, the infrared light emitted by the ray sensor 40 is irradiated on a structure that has a relatively large distance, a relatively weak reflective property, or relatively strong absorption for the infrared light. The reflected infrared light is processed in the same manner as in the first state, and the comparator compares the intensity of the reflected infrared light with the intensity threshold. A signal lower than the intensity threshold is set to 0 and corresponds to the second state of the trigger assembly 5. Thus, the first state and the second state of the trigger assembly 5 are smoothly distinguished.

Referring to FIGS. 2 and 3, in some embodiments of the present application, the trigger assembly 5 includes the trigger sleeve 50 and a reset member 51. The trigger sleeve 50 is slidably connected in the sterile barrier 3, and the reset member 51 abuts against the trigger sleeve 50 and the sterile barrier 3 separately. The laparoscope 6 mounted in place is capable of pressing against the trigger sleeve 50 to move the trigger sleeve 50 so that the trigger sleeve 50 blocks the state detector 4 or a different position of the trigger sleeve 50 faces the state detector 4. The trigger assembly 5 is in the first state so that the state detector 4 receives the infrared light reflected by the trigger sleeve 50 and having an intensity greater than the intensity threshold. When the laparoscope 6 is not mounted in place, the trigger sleeve 50 makes the trigger assembly 5 in the second state under the action of the reset member 51 so that the state detector 4 receives the reflected infrared light whose intensity is lower than the intensity threshold.

For example, the trigger sleeve 50 is slidably connected within the sterile mounting cavity, a cavity for the laparoscope 6 to penetrate through is formed inside the trigger sleeve 50, an upper end face of the trigger sleeve 50 abuts against the laparoscope 6 to move with the laparoscope 6, and the trigger sleeve 50 presses against the reset member 51 so that the reset member 51 deforms elastically. The reset member 51 is a spring sleeved on the outer circumference of the trigger sleeve 50. The spring is in a compressed state when the trigger sleeve 50 is in the first state. The spring is in a natural state when the trigger sleeve 50 is in the second state. It is to be understood that the reset member 51 may be in other elastic structural forms which enable the trigger sleeve 50 to change between the first state and the second state.

After the laparoscope 6 is mounted in place, the trigger sleeve 50 is in the first state, and the infrared light generated by the state detector 4 is directly irradiated on an outer sidewall of the trigger sleeve 50 to form the reflected infrared light whose intensity is greater than the intensity threshold. When the trigger sleeve 50 is not in the first state, since the moving trigger sleeve 50 synchronously compresses the reset member 51, the reset member 51 limits a position of the trigger sleeve 50 so that the trigger sleeve 50 remains in the second state. Since the trigger sleeve 50 moves under the pressing of the laparoscope 6, when the trigger sleeve 50 is in the second state, it indicates that the laparoscope 6 is not mounted in place.

When the laparoscope 6 is not mounted in place, the infrared light emitted by the state detector 4 is irradiated on another reflective surface or reflective structure. Due to a change in the distance or reflective property, the intensity of the reflected infrared light is lower than the intensity threshold. Thus, the position of the trigger sleeve 50 can be smoothly determined, and whether the laparoscope 6 is mounted in place can be determined.

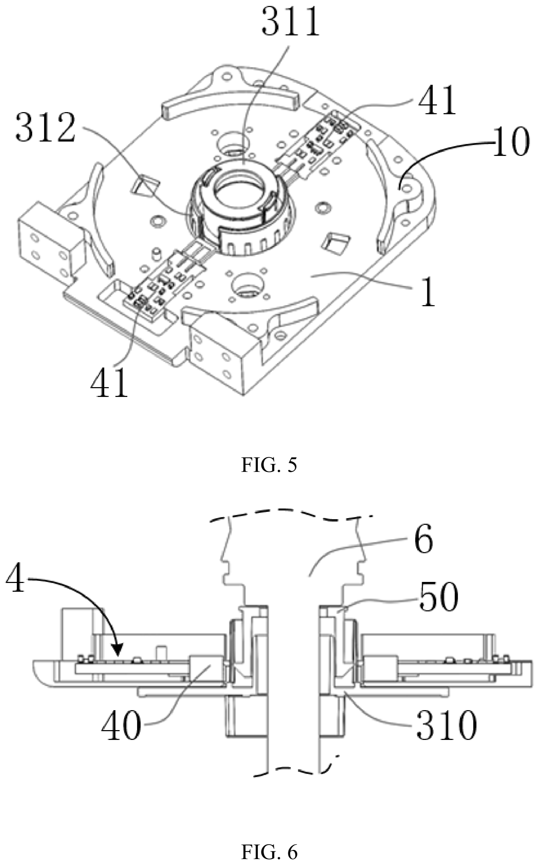

Referring to FIGS. 2 and 5, in some embodiments of the present application, the sterile barrier 3 includes a first sterile shell 30 and a second sterile shell 31. The trigger sleeve 50 is disposed in the first sterile shell 30, and the second sterile shell 31 is partially inserted into the trigger sleeve 50; in the first state, the outer sidewall of the trigger sleeve 50 corresponds to the state detector 4; and in the second state, an outer sidewall of the second sterile shell 31 corresponds to the state detector 4.

For example, the first sterile shell 30 is inserted from an upper side of the drive unit 2, the trigger sleeve 50 is located at an end of the first sterile shell 30 inserted into the drive unit 2, a lower end of the trigger sleeve 50 may extend out of the first sterile shell 30, and a clasp is formed on the outer sidewall of the trigger sleeve 50, where the clasp can be clamped with a lower end of the first sterile shell 30 to limit the position of the trigger sleeve 50.

The second sterile shell 31 includes a sterile plate 310, a sterile sleeve 311, and a top ring 312, where the sterile sleeve 311 and the top ring 312 are both fixed on a side surface of the sterile plate 310, and the top ring 312 is disposed on an outer side of the sterile sleeve 311. The sterile sleeve 311 is inserted into the trigger sleeve 50 from an end of the base plate 1 facing away from the first sterile shell 30, and the top ring 312 is inserted into and fitted to the first sterile shell 30. A detection gap 313 corresponding to the state detector 4 is formed through the top ring 312 so that the state detector 4 can correspond to the sterile sleeve 311. The trigger sleeve 50 can be inserted between the sterile sleeve 311 and the top ring 312 to block the state detector 4. Multiple state detectors 4 may be provided, the multiple state detectors 4 are distributed at intervals along a circumferential direction of the trigger sleeve 50, and detection gaps 313 are correspondingly formed on the top ring 312. In this embodiment, two state detectors 4 are provided and located on two sides of the trigger sleeve 50 respectively, and detection gaps 313 are correspondingly formed on two opposite sides of the top ring 312 respectively.

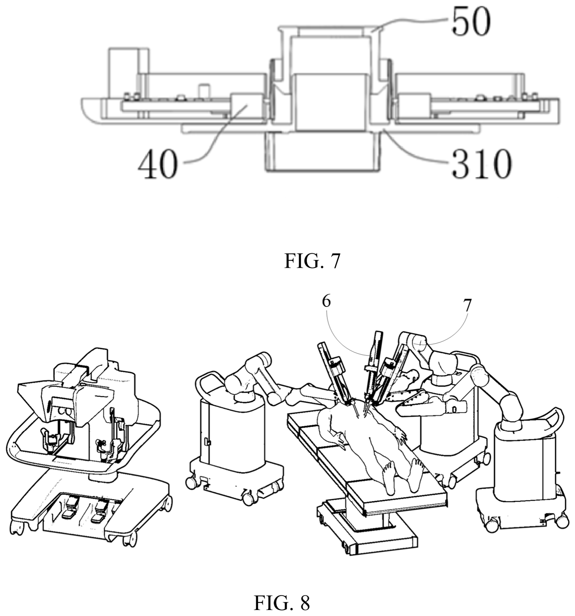

Referring to FIGS. 6 and 7, when the sterile barrier 3 is mounted, the first sterile shell 30 needs to be inserted from the upper side of the drive unit, and then the second sterile shell 31 is inserted from a lower side of the drive unit 2 and inserted into and fitted to the first sterile shell 30. The sterile plate 310 may be provided with structures such as snap-fits and clamped with and fixed to the base plate 1 so that the sterile barrier 3 is quickly mounted in place.

When the sterile barrier 3 is mounted in place, the state detector 4 corresponds to the sterile sleeve 311 through the detection gap 313. When the laparoscope 6 is mounted, the laparoscope 6 abuts against the trigger sleeve 50 to push the trigger sleeve 50 so that the trigger sleeve 50 is gradually inserted between the top ring 312 and the sterile sleeve 311. After the laparoscope 6 is mounted in place, the trigger sleeve 50 blocks the state detector 4 so that the infrared light generated by the state detector 4 is irradiated on the trigger sleeve 50. Compared with the infrared light reflected by the sterile sleeve 311, the infrared light reflected by the trigger sleeve 50 has an intensity greater than the intensity threshold due to a smaller distance between the trigger sleeve 50 and the state detector 4, indicating that the trigger sleeve 50 is in the first state under the pressing of the laparoscope 6 and the laparoscope 6 has been mounted in place.

However, if the second sterile shell 31 is inserted first, the first sterile shell 30 cannot be mounted in place. At this time, even if the laparoscope 6 is inserted into the second sterile shell 31, the trigger sleeve 50 cannot be pushed into movement to make the trigger assembly 5 in the first state so that the state detector 4 may fail to start normally or can only detect the second state of the trigger assembly 5 after startup, indicating that the laparoscope 6 is not mounted in place.

Since the trigger sleeve 50 and the sterile sleeve 311 are both rotary bodies, when the ray sensor 40 is used, the trigger sleeve 50 and the sterile sleeve 311 reflect light at a greater angle than a plane, and the reflected light has a smaller intensity. To ensure that the infrared light reflected by the trigger sleeve 50 in the first state has a relatively large intensity, the distance between the ray sensor 40 and a reflective surface when the infrared light reflected by the reflective surface and received by the ray sensor 40 reaches a peak intensity may be measured in advance, and the distance between the outer sidewall of the trigger sleeve 50 and the infrared photoelectric sensor in the first sate is set to this measured distance. Meanwhile, the distance between an outer sidewall of the sterile sleeve 311 and the ray sensor 40 is set as large as possible, for example, the thickness of the sterile sleeve 311 may be reduced. Thus, the reflected infrared light has significantly different intensities when the trigger sleeve 50 is in the first state and the second state, thereby facilitating the setting of the intensity threshold and helping to distinguish the first state from the second state of the trigger sleeve 50.

In some embodiments of the present application, the outer sidewall of the trigger sleeve 50 has a reflective coating. The second sterile shell 31 is made of a material capable of absorbing the infrared light.

For example, the outer sidewall of the trigger sleeve 50 may be configured with a diffuse reflective surface, and the reflective coating may be formed through a technique such as gold plating, silver plating, or molybdenum plating so that the outer sidewall of the trigger sleeve 50 has a relatively strong reflective property. The sterile sleeve 311 of the second sterile shell 31 may be made of conventional engineering plastic such as polycarbonate (PC), polymethyl methacrylate (PMMA), or acrylonitrile butadiene styrene (ABS) plastic, which has relatively strong absorption for the infrared light. Either or both of the above two means may be used, so as to increase a difference between the intensities of the reflected infrared light when the trigger sleeve 50 is in the first state and the second state.

In some embodiments of the present application, the sterile barrier 3 further includes a mounting detector. The mounting detector is disposed on the base plate 1 to detect a position of the second sterile shell 31 relative to the base plate 1 and is communicatively connected to the state detector 4 via a control module.

For example, insertion blocks are provided on an edge of the sterile plate 310, gaps for the insertion blocks to be inserted through are defined on a bottom wall of the base plate 1, the insertion blocks are clamped with the gaps through rotation, and the rotated sterile plate 310 is fixed by a plunger on the base plate 1 so that the sterile plate 310 is relatively fixed and connected to the base plate 1. The mounting detector may be a photoelectric sensor, and an insertion block blocks the photoelectric sensor when clamped with a gap through rotation, so as to determine whether the sterile plate 310 is mounted in place. When the mounting detector detects the insertion block, the control module controls, according to mounting information, the state detector 4 to restart to start detecting whether the laparoscope 6 is mounted in place. When the mounting detector does not detect the insertion block, the state detector 4 remains closed to avoid false detection.

To achieve more accurate mounting detection, a trigger switch may be provided to control the mounting detector to start, and the trigger switch may be triggered by the second sterile shell 31 inserted into and fitted to the first sterile shell 30. That is, only after the second sterile shell 31 is inserted into and fitted to the first sterile shell 30 and the first sterile shell 30 and the second sterile shell 31 are clamped with and fixed to the drive unit 2, the mounting detector is started to detect whether the second sterile shell 31 is mounted in place.

Referring to FIGS. 1 and 2, the present application provides a laparoscope mounting detection method for detecting a mounted state of a laparoscope mounted in a drive unit 2 via a sterile barrier 3. The laparoscope mounting detection method includes controlling a state detector 4 to start to acquire state information of a trigger assembly 5, in response to the state information satisfying a preset condition, determining that the trigger assembly 5 is in a first state when a laparoscope 6 is mounted in place, and in response to the state information not satisfying the preset condition, determining that the trigger assembly 5 is in a second state when the laparoscope 6 is not mounted in place.

The drive unit 2 has a hollow cylindrical structure, and a drive element is embedded inside the drive unit 2 as a power source. The sterile barrier 3 is detachably connected in the drive unit 2 and connected to the power source, and the power of the power source is used for driving the sterile barrier 3 to rotate. The trigger assembly 5 is disposed in the sterile barrier 3. As the laparoscope 6 is gradually inserted into the sterile barrier 3, the trigger assembly 5 can gradually change from the second state to the first state. The state detector 4 may face the trigger assembly 5 only when the trigger assembly 5 is in the first state, thereby distinguishing the first state from the second state of the trigger assembly 5. Alternatively, the state detector 4 may always face the trigger assembly 5 and can receive different signals in the first state and the second state and distinguish the first state from the second state, where the different signals may be reflected infrared light with different intensities.

According to the laparoscope mounting detection method, when the laparoscope 6 is mounted, the state detector 4 detects the corresponding state information of the trigger assembly 5. When it is detected that the trigger assembly 5 is in the second state, it indicates that the laparoscope 6 is not mounted in place, and when it is detected that the trigger assembly 5 is in the first state, it indicates that the laparoscope 6 is mounted in place. During detection, the state detector 4 is neither in direct contact with the laparoscope 6 nor occupies an axial space of the laparoscope 6 to affect an operating route. Moreover, the state of the trigger assembly 5 only indicates a mounted position of the laparoscope 6 and does not change a rotation structure of the laparoscope 6 so that the laparoscope 6 is still driven to rotate by the drive unit 2 via the sterile barrier 3, thereby maintaining relatively high conversion efficiency, avoiding an increase in the friction between the laparoscope 6 and the sterile barrier 3, and ensuring that the laparoscope 6 is prevented from stuttering during insertion and removal.

In some embodiments of the present application, acquiring the state information of the trigger assembly 5 includes controlling the state detector 4 to emit infrared light and receive infrared light reflected by the trigger assembly 5 to determine the state information of the trigger assembly 5 according to an intensity of the reflected infrared light.

For example, the infrared light emitted by the state detector 4 is reflected when irradiated on any structure, and the intensity of the reflected infrared light changes due to factors such as the distance and absorption and reflection of the infrared light by the irradiated structure. Therefore, when the trigger assembly 5 gradually changes from the second state to the first state, the intensity of the reflected infrared light may change as long as any one of the above three factors changes, and the state information of the trigger assembly 5 can be determined according to the change in the intensity of the infrared light.

As the laparoscope 6 is gradually inserted into the sterile barrier 3, the trigger assembly 5 gradually changes to the first state as the laparoscope 6 moves. The state detector 4 can receive the reflected infrared light and according to the change in the intensity of the reflected infrared light, determine whether the trigger assembly 5 is in the first state, that is, whether the laparoscope 6 is mounted in place.

In some embodiments of the present application, the state information satisfying the preset condition includes: in response to the intensity of the reflected infrared light being greater than an intensity threshold, determining that the trigger assembly 5 is in the first state.

The first state indicates that the laparoscope 6 is mounted in place. The laparoscope 6 is inserted into the sterile barrier 3 during mounting, that is, the laparoscope 6 gradually extends into the sterile barrier 3 during mounting, and the trigger assembly 5 moves synchronously as the laparoscope 6 extends so that the trigger assembly 5 gradually changes from the second state to the first state.

As the trigger assembly 5 moves, the trigger assembly 5 may gradually block the infrared light emitted by the state detector 4 and generate the reflected infrared light so that the infrared light is reflected at a smaller distance, or the infrared light emitted by the state detector 4 may be irradiated on a coating with a stronger reflective property so that the intensity of the reflected infrared light increases. A setting of the intensity threshold only needs to be greater than the intensity of the reflected infrared light when the trigger assembly 5 is in the second state.

According to the preceding method, when the intensity of the reflected infrared light received by the state detector 4 is greater than the intensity threshold, it may be determined that the reflected infrared light is generated by being irradiated on the trigger assembly 5 in the first state so that it can be determined that the trigger assembly 5 is in the first state.

In some embodiments of the present application, before the state detector 4 is controlled to start, the laparoscope mounting detection method further includes: acquiring mounting information of the sterile barrier 3, and in response to the mounting information satisfying a set condition, controlling the state detector 4 to start.

The state detector 4 is configured to detect the trigger assembly 5, and the trigger assembly 5 is disposed in the sterile barrier 3. Therefore, before the sterile barrier 3 is mounted in place, the state detector 4 needs to remain closed to avoid false detection. For the mounting detection of the sterile barrier 3, one detection element may be separately provided, and the detection element is communicatively connected to the state detector 4 via a control module. The detection element can detect a mounting situation of the sterile barrier 3 to generate the mounting information. When it is determined that the sterile barrier 3 is mounted in place, the control module controls the state detector 4 to start correspondingly.

Through the above steps, the state detector 4 remains closed before the sterile barrier 3 is mounted in place, so as to avoid false detection. After the sterile barrier 3 is mounted in place, the state detector 4 automatically starts according to the corresponding mounting information so that the whole detection process is more automated, and an operation process can be effectively simplified, making it convenient to quickly mount the laparoscope 6.

In some embodiments of the present application, the mounting information satisfying the set condition includes, for example, mounting a first sterile shell 30 prior to a second sterile shell 31.

Since the sterile barrier 3 includes the first sterile shell 30 and the second sterile shell 31, the mounting information refers to mounting information of the first sterile shell 30 and the second sterile shell 31, and the preset condition refers to information that both the first sterile shell 30 and the second sterile shell 31 are mounted in place.

An end of the first sterile shell 30 inserted into the drive unit 2 has snap-fit structures clamped with the drive unit 2, and the snap-fit structures can generate certain deformation to facilitate the smooth insertion of the first sterile shell 30. The second sterile shell 31 may prevent the deformation of the snap-fit structures after being inserted into the first sterile shell 30 so that the first sterile shell 30 is clamped and fixed to the drive unit 2. The second sterile shell 31 may be fixed to the base plate 1 through its own locking structures.

Therefore, when the sterile barrier 3 is mounted, the first sterile shell 30 has to be inserted prior to the second sterile shell 31. In this case, when a mounting detector detects a position of the second sterile shell 31 relative to the base plate 1, it may be determined that the sterile barrier 3 is mounted in place, that is, the preset condition is satisfied, and the state detector 4 can automatically start at this moment.

However, when the second sterile shell 31 is inserted first, since the snap-fit structures of the first sterile shell 30 cannot be clamped and fixed to the drive unit 2 through deformation, the first sterile shell 30 cannot be inserted, the second sterile shell 31 cannot be inserted into the first sterile shell 30, and a trigger switch cannot be triggered. Thus, the mounting detector remains closed and fails to detect the mounting information. In this case, the mounting information does not satisfy the preset condition, and the state detector 4 does not start or detect whether the laparoscope 6 is mounted in place, or the state detector 4 can only detect the second state of the trigger assembly 5 after startup.

Even if the mounting detector can be triggered and acquire information that the second sterile shell 31 is mounted in place, the first sterile shell 30 cannot be mounted to a specified depth, and the second sterile shell 31 cannot be inserted into the first sterile shell 30. Then, when the laparoscope 6 is inserted and pushes the trigger assembly 5 into movement, the trigger assembly 5 fails to move to a corresponding depth and trigger the state detector 4 to perform the corresponding detection.

The present application provides a surgical robot including a robotic arm, a laparoscope 6, and the laparoscope drive system according to any preceding embodiment. The laparoscope drive system is disposed on the robotic arm and connected to the laparoscope 6.

During application of the surgical robot, the laparoscope drive system can not only effectively detect a mounted state of the laparoscope 6 during mounting of the laparoscope 6 but also ensure that an operating route and the rotary action of the laparoscope 6 are not affected, thereby preventing the laparoscope 6 from stuttering during insertion and removal and effectively ensuring the overall usability and operation experience of the surgical robot.

### Embodiment two

This embodiment is based on embodiment one and differs from embodiment one in that the trigger sleeve 50 has a different composition.

In some embodiments of the present application, the trigger sleeve 50 includes a first collar and a second collar 502 distributed in a stepped shape; in the first state, an outer sidewall of the first collar 501 corresponds to the state detector 4; and in the second state, an outer sidewall of the second collar 502 corresponds to the state detector 4.

For example, the first collar 501 is located above the second collar 502, the diameter of the first collar 501 is smaller than that of the second collar 502, and the trigger sleeve 50 may have the same inner diameter to ensure the smooth mounting of the laparoscope 6. The sterile sleeve 311 is inserted into the trigger sleeve 50, and the second collar 502 is located between the state detector 4 and the sterile sleeve 311.

When the laparoscope 6 is inserted, the laparoscope 6 abuts against an end face of the first collar 501 and can push the trigger sleeve 50 into movement so that the first collar 501 corresponds to the state detector 4, and the infrared light emitted by the state detector 4 is irradiated on the outer sidewall of the first collar 501. A reflective surface that reflects the infrared light changes from the second collar 502 with the larger diameter to the first collar 501 with the smaller diameter, the reflection distance increases, and the reflected intensity changes. It may be set that the distance between the first collar 501 and the state detector 4 can yield a peak intensity of the reflected infrared light. Of course, it may be set that the distance between the second collar 502 and the state detector 4 can yield the peak intensity of the reflected infrared light. Alternatively, the outer sidewall of the first collar 501 and the outer sidewall of the second collar 502 have coatings with different reflective properties, or the first collar 501 and the second collar 502 may be made of materials with different infrared light absorption properties, so as to increase a difference between intensities of the reflected infrared light.

### Embodiment three

This embodiment is based on embodiment one and differs from embodiment one in that the trigger sleeve 50 has a different structure.

In some embodiments of the present application, an outer surface of the trigger sleeve 50 is coated with a first reflective coating and a second reflective coating with different reflective properties; in the first state, the first reflective coating corresponds to the state detector 4; and in the second state, the second reflective coating corresponds to the state detector 4.

For example, the trigger sleeve 50 is in the shape of a hollow cylinder, and the lower end of the trigger sleeve 50 extends directly between the sterile sleeve 311 and the top ring 312 and blocks the state detector 4. The first reflective coating and the second reflective coating are both coated on the outer sidewall of the trigger sleeve 50 so that the trigger sleeve 50 can be divided into an upper part and a lower part with the same diameter. It is to be understood that one of the first reflective coating and the second reflective coating may be replaced with a coating that can absorb the infrared light, and the other is a coating that can reflect the infrared light so that the intensity of the reflected infrared light can also change. For example, the properties of the first reflective coating and the second reflective coating may be designed according to actual machining conditions, which are not limited in the present application.

In the first state, the first reflective coating on the upper part reflects the infrared light generated by the state detector 4, and in the second state, the second reflective coating on the lower part reflects the infrared light generated by the state detector 4, so as to represent the first state and the second state of the trigger sleeve 50. It is to be understood that multiple reflective coatings may be provided, and reflective properties of the multiple reflective coatings may gradually increase or decrease from top to bottom along an axial direction of the trigger sleeve 50 so that as the trigger sleeve 50 gradually slides down, different reflective coatings correspond to the state detector 4, the state detector 4 can acquire reflected infrared light with different intensities, and multiple intensities of the reflected infrared light can show an increase or decrease trend, so as to accurately determine a state of the trigger sleeve 50, which can more accurately indicate the mounted state of the laparoscope 6.

## Claims

1. A laparoscope drive system, comprising:
a base plate (1);
a drive unit (2) disposed on the base plate (1) and configured to drive a laparoscope (6) to rotate via a sterile barrier (3);
a trigger assembly (5) disposed in the sterile barrier (3) and selectively abutting against the laparoscope (6), wherein the trigger assembly (5) has a first state indicating that the laparoscope (6) is mounted in place and a second state indicating that the laparoscope (6) is not mounted in place; and
a state detector (4) disposed in the base plate (1) and configured to detect state information of the trigger assembly (5) to determine a mounted state of the laparoscope (6).

2. The laparoscope drive system according to claim 1, wherein the trigger assembly (5) comprises:
a trigger sleeve (50) slidably connected in the sterile barrier (3); and
a reset member (51) abutting against the trigger sleeve (50) and the sterile barrier (3) separately;
wherein the laparoscope (6) mounted in place is capable of pressing against the trigger sleeve (50) so that the trigger assembly (5) is in the first state, and when the laparoscope (6) is not mounted in place, the trigger sleeve (50) makes the trigger assembly (5) in the second state under the action of the reset member (51).

3. The laparoscope drive system according to claim 2, wherein the state detector (4) is capable of generating a ray and receiving a ray reflected by the sterile barrier (3) or the trigger sleeve (50) to detect the state information of the trigger assembly (5) according to an intensity of the reflected ray.

4. The laparoscope drive system according to claim 3, wherein the sterile barrier (3) comprises a first sterile shell (30) and a second sterile shell (31), the trigger sleeve (50) is disposed in the first sterile shell (30), and the second sterile shell (31) is partially inserted into the trigger sleeve (50); in the first state, an outer sidewall of the trigger sleeve (50) corresponds to the state detector (4); and in the second state, an outer sidewall of the second sterile shell (31) corresponds to the state detector (4).

5. The laparoscope drive system according to claim 4, wherein at least one of the following is satisfied:
the outer sidewall of the trigger sleeve (50) has a reflective coating; or
the second sterile shell (31) is made of a material capable of absorbing the ray.

6. The laparoscope drive system according to claim 3, wherein the trigger sleeve (50) comprises a first collar (501) and a second collar (502) distributed in a stepped shape; in the first state, an outer sidewall of the first collar (501) corresponds to the state detector (4); and in the second state, an outer sidewall of the second collar (502) corresponds to the state detector (4).

7. The laparoscope drive system according to claim 3, wherein an outer surface of the trigger sleeve (50) is coated with a first reflective coating and a second reflective coating which have different reflective properties; in the first state, the first reflective coating corresponds to the state detector (4); and in the second state, the second reflective coating corresponds to the state detector (4).

8. The laparoscope drive system according to any one of claims 1 to 7, wherein the state detector (4) comprises:
a ray sensor (40) configured to emit the ray and receive the reflected ray;
a detection circuit board (41) communicatively connected to the ray sensor (40) to convert the reflected ray into an analog signal;
an amplifier disposed on the detection circuit board (41) and configured to amplify the analog signal; and
a comparator disposed on the detection circuit board (41) and communicatively connected to the amplifier to perform comparative processing on the amplified analog signal to generate the corresponding state information.

9. The laparoscope drive system according to any one of claims 1 to 7, further comprising:
a mounting detector disposed on the base plate (1), communicatively connected to the state detector (4) via a control module, and configured to detect a position of the sterile barrier (3) relative to the base plate (1).

10. A laparoscope mounting detection method, comprising:
controlling a state detector (4) to start to acquire state information of a trigger assembly (5), in response to the state information satisfying a preset condition, determining that the trigger assembly (5) is in a first state when a laparoscope (6) is mounted in place, and in response to the state information not satisfying the preset condition, determining that the trigger assembly (5) is in a second state when the laparoscope (6) is not mounted in place.

11. The laparoscope mounting detection method according to claim 10, wherein controlling the state detector (4) to start to acquire the state information of the trigger assembly (5) comprises:
controlling the state detector (4) to emit a ray and receive a ray reflected by the trigger assembly (5) to determine the state information of the trigger assembly (5) according to an intensity of the reflected ray.

12. The laparoscope mounting detection method according to claim 11, wherein the state information satisfying the preset condition comprises:
in response to the intensity of the reflected ray being greater than an intensity threshold, determining that the trigger assembly (5) is in the first state.

13. The laparoscope mounting detection method according to claim 10, before controlling the state detector (4) to start, further comprising:
acquiring mounting information of a sterile barrier (3), and in response to the mounting information satisfying a set condition, controlling the state detector (4) to start.

14. The laparoscope mounting detection method according to claim 13, wherein the mounting information satisfying the set condition comprises:
mounting a first sterile shell (30) prior to a second sterile shell (31).

15. A surgical robot, comprising a robotic arm (7) and a laparoscope (6) and further comprising the laparoscope drive system according to any one of claims 1 to 9, wherein the laparoscope drive system is disposed on the robotic arm (7) and drivingly connected to the laparoscope (6).
